Europäisches Patentamt

European Patent Office

Office européen des brevets

(11) Publication number: **0 120 054**
**B1**

# EUROPEAN PATENT SPECIFICATION

(45) Date of publication of patent specification: **30.05.90**

(51) Int. Cl.⁵: **C 07 D 209/58, A 61 K 31/40**

(21) Application number: **83903067.3**

(22) Date of filing: **09.09.83**

(86) International application number:
**PCT/US83/01379**

(87) International publication number:
**WO 84/01382 12.04.84 Gazette 84/10**

(54) **PURIFIED HEMATOPORPHYRIN DERIVATIVE FOR DIAGNOSIS AND TREATMENT OF TUMORS, AND METHOD.**

(30) Priority: **27.09.82 US 424647**
**01.04.83 US 481345**

(43) Date of publication of application:
**03.10.84 Bulletin 84/40**

(45) Publication of the grant of the patent:
**30.05.90 Bulletin 90/22**

(84) Designated Contracting States:
**AT BE CH DE FR GB LI NL SE**

(56) References cited:
**CHEMICAL ABSTRACTS, vol. 97, no. 1, July 5, 1982, page 394, abstract 3930s, COLUMBUS, OHIO (US), SHILIN XU et al.: "Hematoporphyrin sodium, as a chemotherapeutic agent", & Zhongcaoyao 1981, 12(8), 343-4.**

**The file contains technical information submitted after the application was filed and not included in this specification**

(73) Proprietor: **PHOTOFRIN MEDICAL INC.**
**Suite 952 NBC Center**
**Lincoln,Nebraska 68508 (US)**

(72) Inventor: **WEISHAUPT, Kenneth, R.**
**94 Rutland Avenue**
**Sloan, NY 14212 (US)**
Inventor: **DOUGHERTY, Thomas, J.**
**2306 West Oakfield**
**Grand Island, NY 14072 (US)**
Inventor: **POTTER, William, R.**
**1876 East River Road**
**Grand Island, NY 14072 (US)**

(74) Representative: **UEXKÜLL & STOLBERG**
**Patentanwälte**
**Beselerstrasse 4**
**D-2000 Hamburg 52 (DE)**

EP 0 120 054 B1

Courier Press, Leamington Spa, England.

# EP 0 120 054 B1

(56) References cited:

CHEMICAL ABSTRACTS, vol. 78, no. 11, March 19, 1973, page 212, abstract 68960d, COLUMBUS, OHIO (US),D.R. SANDERSON et al.: "Hematoporphyrin as a diagnostic tool. Preliminary report of new techniques".& Cancer (Philadelphia) 1972, 30(5), 1368-72.

CHEMICAL ABSTRACTS, vol. 98, no. 9, February 28, 1983, page 293, abstract 68090h, COLUMBUS, OHIO (US), C.J. GOMER et al.: "Hematoporphyrin derivative photoradiation therapy for the treatment of intraocular tumors; examination of acute normal ocular tiss Dougherty et al., Journal National Cancer Institute, vol. 62, pp. 231-37 (1979) Dougherty et al., Cancer Research, vol. 38 pp.2628-2635 (1978)

Lipson et al., Journal National Cancer Institut, vol. 26, pp. 1-8 (1961)

## Description

This invention relates to photosensitive compounds useful in diagnosis and methods of preparing them.

It is known in the prior art to diagnose malignant tumors with photosensitive drugs. In "The Use of a Derivative of Hematoporphyrin in Tumor Detection", J. Natl Cancer Inst. 26:1—8, 1961, Lipson and co-workers disclosed a crude material, prepared by acetic acid-sulfuric acid treatment of hematoporphyrin, said material having a superior ability to localize in tumors. The photosensitive characteristic of tumor-selective porphyrin compounds also make them useful in the treatment of tumors.

In "Photoradiation Therapy for the Treatment of Malignant Tumors", Cancer Res. 38: 2628—2635, 1978 and "Photoradiation in the Treatment of Recurrent Breast Carcinoma", J Natl Cancer Inst. 62: 231—237, 1979, Dougherty and co-workers reported using the crude Lipson hematoporphyrin derivative to accomplish photoradiation therapy on human patients. The crude Lipson hematoporphyrin derivative has the ability to enter all kinds of cells and to be retained in tumor cells after it has mostly cleared the serum. Subsequent irradiation with red light excites the crude Lipson derivative which in turn excites oxygen molecules. The excited oxygen molecules exist for a microsecond — long enough to attach to tumor cell walls and effect necrosis.

The crude Lipson hematoporphyrin derivative has the disadvantage of entering normal tissue and causing unacceptable damage when therapeutic light sufficient to treat large tumors is applied. Severe edema and sloughing of healthy skin can occur when the crude Lipson derivative is used. Some patients are even harmed by exposure to ordinary sunlight thirty days following treatment with the drug. A better photosensitizer for treatment of tumors would have no systemic toxicity.

It is a task of the invention to provide a purified photosensitive substance and method of obtaining it.

The new drug is a substantially pure hematoporphyrin derivative being a high molecular-weight derivative, each molecule of which has an empirical formula of $C_{34} H_{35-36} N_4 O_{5-6} Na_2$. The drug has absorption peaks in the visible spectrum at approximately 505, 537, 565 and 615 nm, with absorption peaks in the infrared spectrum at approximately 3.0, 3.4, 6.4, 7.1, 8.1, 9.4, 12 and 15 µm (microns), adsorption peaks in carbon-13 nuclear magnetic resonance study at approximately 9.0, 18.9, 24.7, 34.5, 62, 94.5, 130—145, 171.7 ppm and possibly 118 and 127 relative to a 37.5 ppm resonance peak of dimethyl sulfoxide. The substance shows mass numbers of 1899, 1866, 1809, 1290, 1200, 609, 591, 219 and 149 according to mass spectroscopy and advantageously is in liquid form having a concentration of approximately 2.5 mg/cc. The hematoporphyrin derivative is obtainable by means of specific process steps as outlined below.

Other advantages of the invention will be apparent from the following description, taken in conjunction with the accompanying drawings.

The problems underlying the present invention are solved by providing a composition of matter effective for localizing and/or destroying tumors, said composition being fluorescent and photosensitive and having the capability of localizing in and being retained in tumor cells as compared to normal tissues, whereby the composition is comprised of high molecular aggregates of a porphyrine derivative, said composition further having a visible spectrum with absorption peaks at 505, 537, 565, and 615 nm, an infrared spectrum with absorption peaks at 3.0, 3.4, 6.4, 7.1, 8.1, 9.4, 12 and 15 µm (microns), a carbon-13 NMR resonance spectrum at 9.0, 18.9, 24.7, 34.5, 62, 94.5, 130—145, and 171.7 ppm relative to a 37.5 ppm resonance for DMSO, and a mass spectrum showing mass numbers of 1899, 1866, 1809, 1290, 1200, 609, 591, 219, and 149 obtainable by

a) reacting hematoporphyrine with acetic/sulfuric acid and precipitating the resulting derivative,

b) adjusting the pH of an aqueous suspension of said derivative to 7 to 7.4 and

c) recovering high molecular aggregates of the derivative by filtration through a membrane system excluding substances with a molecular weight below 10,000.

Description of the Drawings

FIGURE 1 is a mass spectrometry print-out of the new drug;

FIGURE 2 is a visible light spectrum of the new drug in water solution;

FIGURE 3 and FIGURE 3A in combination illustrate an infra red spectrum of the new drug dispersed in potassium bromide;

FIGURE 4 is a carbon-13 nuclear magnetic resonance print-out of the new drug, referenced to dimethyl sulfoxide.

FIGURE 5 and FIGURE 5A in combination illustrate a print-out from a Waters Associates Variable Wave Length Detector used in conjunction with its µm Bondpak C-18 column, showing various components including a peak formation representative of the new drug.

FIGURE 6 and FIGURE 6A in combination illustrate a print-out from a Waters Associates Variable Wave Length Detector used in conjunction with its µm Bondpak C-18 column, showing the peak formation of the new drug, per se.

FIGURE 7 is a molecular formula depicting an ether, most likely the unit which associates to form the high molecular weight aggregate of this invention.

FIGURE 8 and FIGURE 8A in combination illustrate a carbon-13 nuclear magnetic resonance print-out of the new drug, referenced to tetramethylsilane in deuterated chloroform solvent. Magnification of the spectrum is shown in the ranges from 20—30 ppm and 55—75 ppm.

EP 0 120 054 B1

(A) Preparation and Purification of the New Drug. (All equipment and reagents must be sterile.)

Add 285 ML of acetic acid to a 1000 ML Erlenmeyer flask containing a Teflon®-coated magnetic stirring bar. Stir the acetic acid and slowly add 15 ML of concentrated sulfuric acid. Weigh out 15.0 grams of hematoporphyrin hydrochloride (preferably obtained from Roussel Corporation, Paris, France) and add said porphyrin to the acid solution. Stir for one hour.

Prepare a solution of 150 grams of sodium acetate in 3 liters of glass-distilled water using a 4-liter glass beaker. At the end of one hour, filter the porphyric-acid solution, preferably through Whatman No. 1 filter paper, allowing the filtrate to drip into the 4-liter beaker of 5% sodium acetate. The 5% sodium acetate solution now contains a dark red precipitate which is preferably allowed to stand for one hour with occasional stirring. The dark red precipitate is then again filtered, preferably using the above-identified filter mechanism. The filter cake from the filtering process is then washed with glass-distilled water until the filtrate is at pH 5.5—6.0 (1500—2500 ML of wash water may be required). The filter cake is then preferably allowed to dry in air at room temperature.

The air-dried precipitate is ground, using for instance, a mortar and pestle until a fine powder is obtained. The powder may then be transferred to 250 ML round bottom flask. The flask is then attached to a rotating evaporator and rotation under vacuum is maintained at room temperature for preferably 24 hours.

20.00 grams of the vacuum-dried powder is then preferably placed in a 4-liter aspirator bottle which may contain a magnetic stirring bar, and then 1000 ML of 0.1N sodium hydroxide is added thereto. This solution is preferably stirred for one hour and 1N hydrochloric acid is then added dropwise preferably using a buret. The 1N HCL is added until a pH of 7.0—7.4 is obtained and which is stable for 15 minutes. Using for instance the buret readings, the amount of sodium chloride produced from the neutralization of sodium hydroxide by the hydrochloric acid may be calculated.

Isotonic solution is 0.9% NaCl or 9 grams NaCl per liter of solution. Therefore, the amount of NaCl produced during neutralization is subtracted from the amount of NaCl required to make the solution isotonic. The calculated amount of NaCl is then added to the solution, and the solution is stirred for preferably 15 minutes. The quantity of solution should then be brought to a total volume of 4 liters by adding 0.9% NaCl solution.

The aspirator bottle, containing the said solution, is then attached to transfer lines leading to a Milli-Pore Pellicon Cassette system fitted with a 10,000 molecular weight filter pack (Millipore Corporation, Bedford, Mass. 01730). It is preferable that the pH of the solution be 7.0—7.2 during this filtration process, and it is preferable that the temperature of the solution be ambient. The Pellicon cassette system should preferably contain at least 25 liters of isotonic saline solution.

The peristalic feed pump is turned on and the solution is run through the Pellicon cassette system at a pressure of preferably 0.69—1,38 bar (10—20 p.s.i.g.). Pressure may be varied depending on the flow rate through the system. Saline is added to the system to maintain a volume of 4 liters in the associated aspirator bottle containing the solution.

The filtration process is continued until the solution contains substantially only the high molecular weight, biologically active product. At this time waste monomers are generally no longer present. Exclusion of the waste through the microporous membrane of the filter system is confirmed by analyzing the high molecular weight, biologically active product with a Bio-Gel P-10® column (obtainable for instance from Bio-Rad, Richmond, Ca.) or by high performance liquid chromatography using a μm Bondpak C-18 column with fixed variable wave length detector (obtainable for instance from Waters Associates, Milford, Ma.), as will be hereinafter described.

Concentrations of the product may be increased by running the Pellicon cassette system without saline feed. Concentrations of the product may be decreased by adding saline solution. It is preferable that concentration of the new drug in solution is approximately 2.5 mg/cc.

(B) Animal Tests of the New Drug

$DBA_2$ Ha/D mice were transplanted with SMT—F tumors. When the transplanted tumors reached 5—6 mm in diameter, the mice were injected with a dose of 7.5 milligrams of the crude prior art Lipson derivative per kilogram of body weight for comparison purposes. Approximately 24 hours following the injection, the tumor areas of the mice were shaved to remove the fur. The mice were exposed to red light (600—700 nm) (6000—7000 Å) from an arc lamp at an intensity of 160 milliwatts per square centimeter for 30 minutes. Ten of twenty mice showed no apparent tumors seven days after treatment. The injected drug is retained in the tumor cells longer as compared to normal tissue. This protocol was repeated using the new drug disclosed in this invention and equivalent results were obtained but using a drug dose of approximately only one-half (4 mg/kg of body weight), as compared to the prior art Lipson drug. Red light is used in treating tumors by illuminating the same in order to take advantage of the ability of the longer visible wave lengths to penetrate tissue. Illumination may be accomplished by direct illumination or by fiber optics, thus providing for illumination of any portion of the body generally without surgery.

In further tests ICR Swiss (Albino) mice were injected with a therapeutic dose of the crude Lipson derivative (7.5 mg/kg of body weight). Approximately 24 hours following such injection, the hind feet of the mice were exposed to the same light conditions used in the aforedescribed tumor response study. The damage to the hind feet was assessed as 2.0 on an arbitrary scale where 0.0 is no damage and 5.0 is complete necrosis. Moist desquamation was evident and the foot area slowly returned to normal after

4

approximately 40 days. This protocol was repeated using the new drug disclosed in this application in doses of 4 mg/kg of body weight. Only slight erythema and/or edema was noticed following treatment. This condition disappeared after 48—72 hours with no residual effects. This leads us to believe that skin photosensitivity may no longer be a significant problem when using this new drug.

(C) Analysis of the Drug

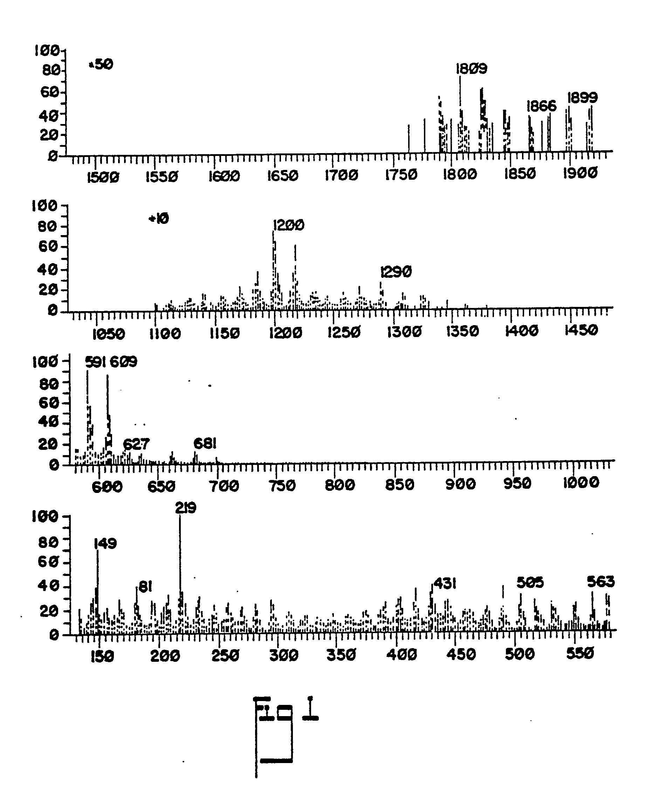

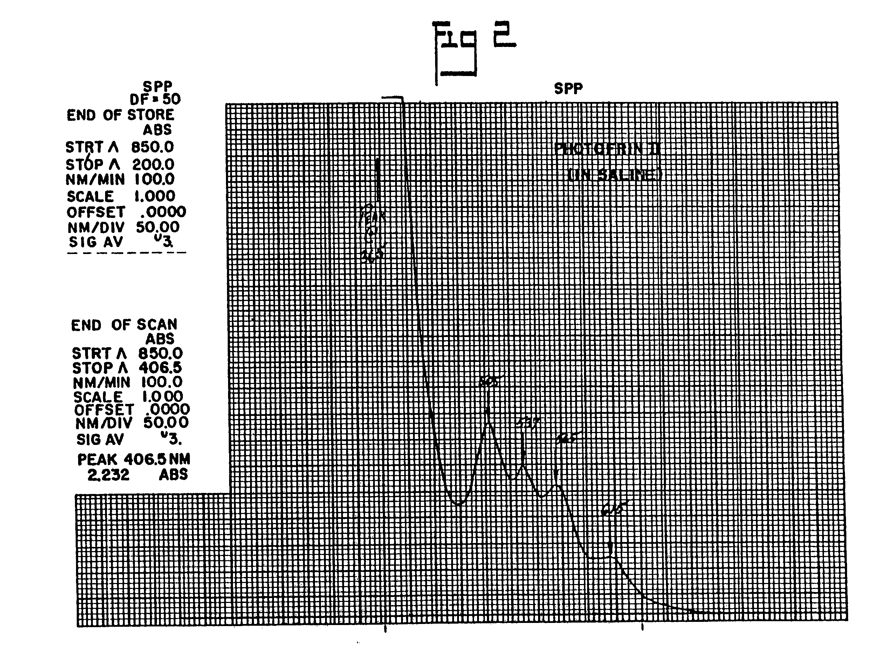

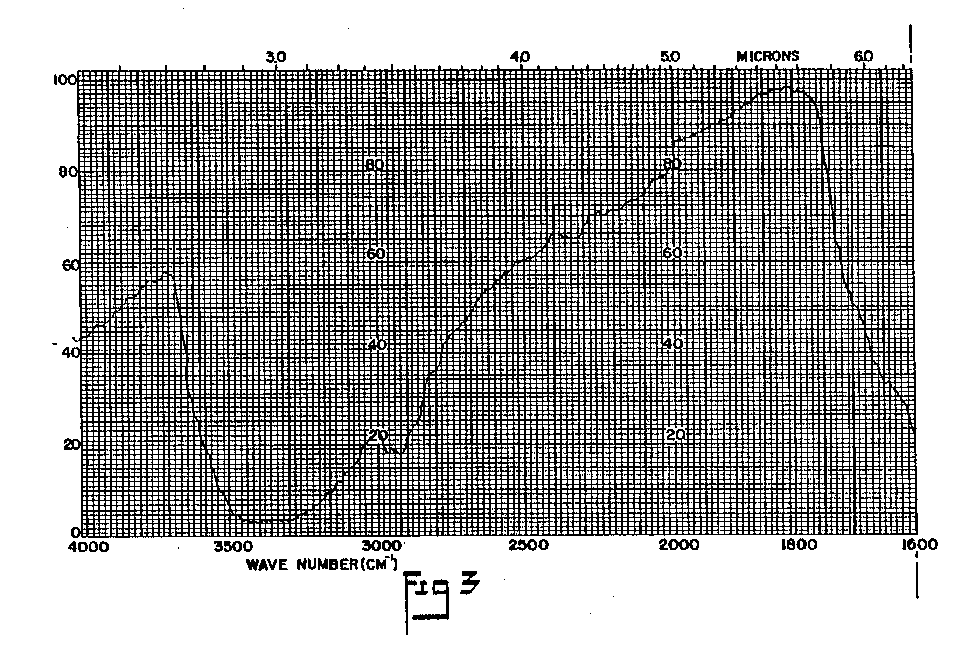

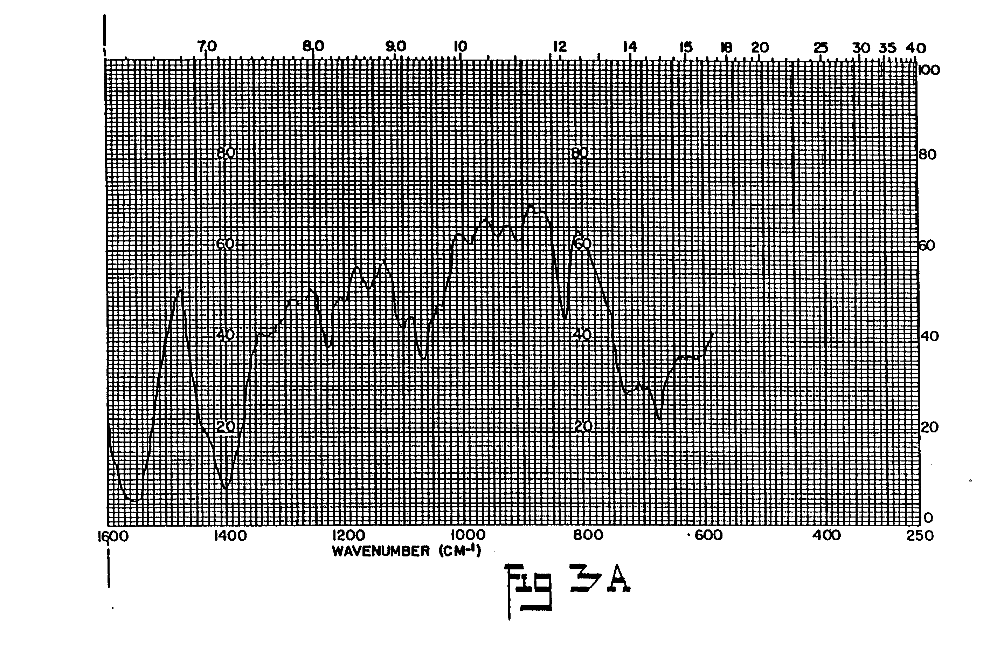

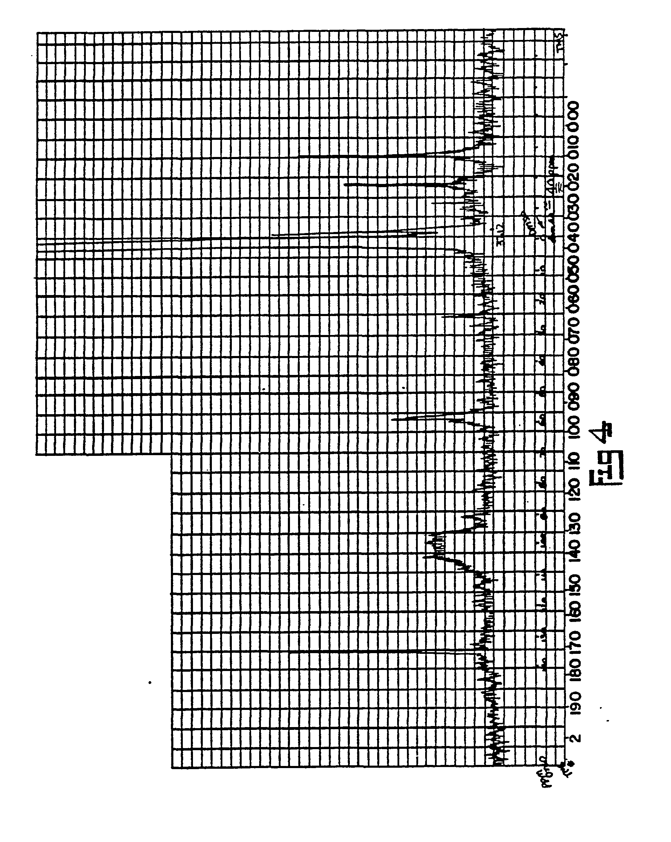

This new drug, as obtained from the Pellicon system, is a high molecular weight material derived by treating hematoporphyrin hydrochloride with acetic and sulfuric acids followed by appropriate hydrolysis. Its failure to pass through the Milli-Pore Pellicon® 10,000 molecular weight filter pack indicates a molecular weight in excess of ten thousand. Mass spectrometry (FIGURE 1) of the new drug shows especially strong peaks at mass numbers of 149, 219, 591, 609 and characteristic but smaller peaks at 1200, 1218, 1290, 1809.

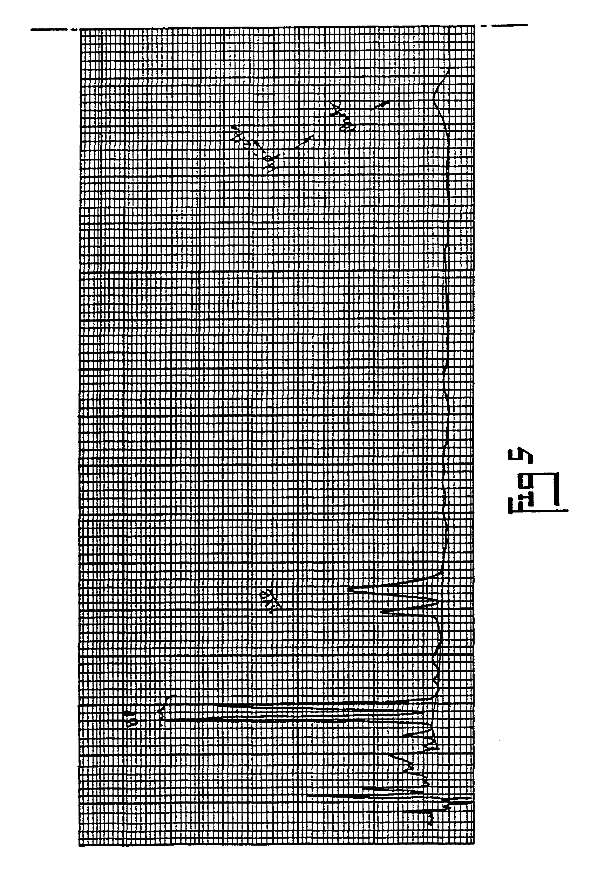

Spectrophotometry (FIGURE 2) of the new orange-red colored drug in aqueous solution reveals well-defined peaks at approximately 505, 537, 565 and 615 millimicrons.

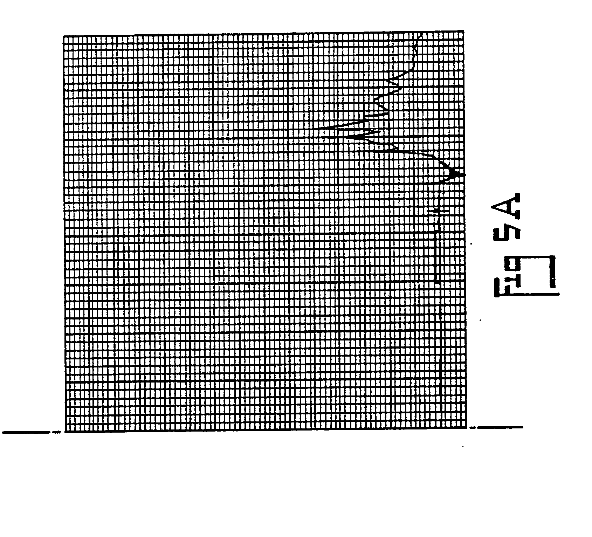

Infrared spectrophotometry (FIGURE 3 and FIGURE 3A) of the new drug disbursed in potassium bromide, reveals a broad peak associated with hydrogen stretching, said peak centered at approximately 3.0 microns, and a shoulder at approximately 3.4 microns. Finer peaks are observed at approximately 6.4, 7.1, 8.1, 9.4, 12 and 15 microns.

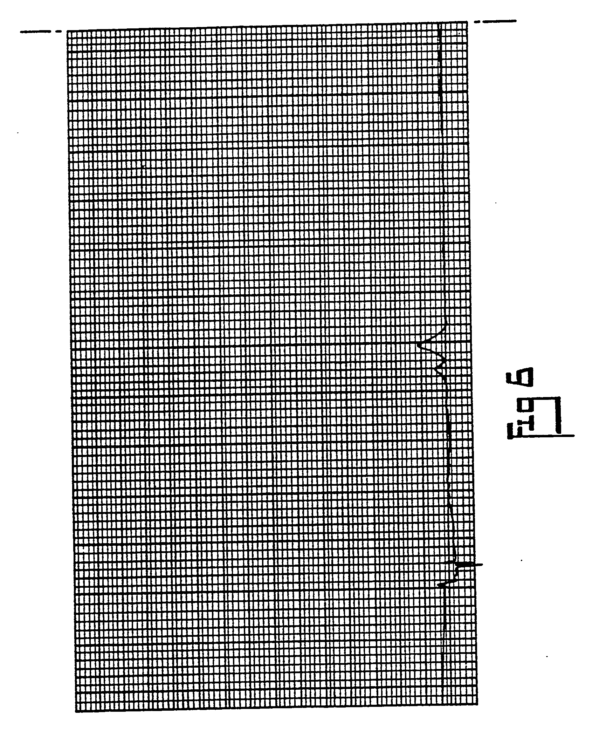

Elemental analysis of the disodium salt derivative of the new drug shows it to have an empirical formula of $C_{34}H_{35-36}N_4O_{5-6}Na_2$, there being some uncertainty in hydrogen and oxygen due to traces of water which cannot be removed from the drug. A carbon-13 nuclear magnetic resonance study (FIGURE 4) of the drug in completely deuterated dimethylsulfoxide shows peaks at approximately 9.0 ppm for —$CH_3$, 18.9 ppm for —$CH_2$—, 24.7 ppm for $CH_3$ CHOH, 34.5 ppm for —$CH_2$—, 62 ppm for $CH_3$ CHOH, 94.5 ppm for =C (methine), 130—145 ppm for ring C, and 171.7 ppm for C≡O, all ppm being relative to dimethyl sulfoxide resonance at about 37.5 ppm. Additional vinyl peaks at approximately 118 and 127 ppm may be representative of the new drug or possibly a contaminant.

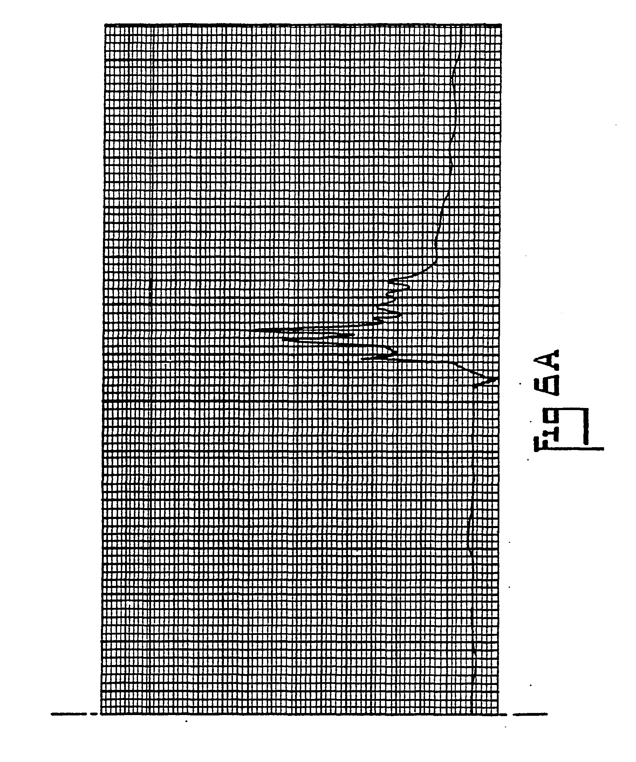

When the unfiltered reaction product described on page 7, lines 4—13 of this application, was eluted from a Waters Associates' μm Bondpak C-18 column using first, successively methanol, water and acetic acid (20:5:1) and then using tetrahydrofuran and water (4:1), four components were found. Three byproducts were identified as hematoporphyrin, hydroxyethylvinyldeuteroporphyrin and protoporphyrin by comparison with standards on thin layer chromatography, with Rf values of approximately 0.19, 0.23, and 0.39 respectively (FIGURE 5) using Brinkman SIL silica plates and benzene-methanol-water (60:40:15) as elutent.

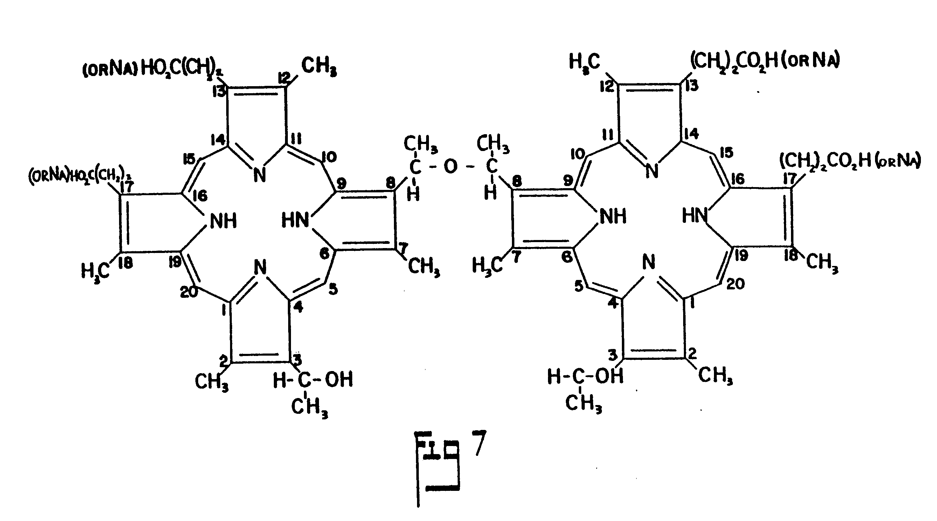

The fourth component shown in FIGURE 5A, was the biologically active drug of the invention. Chromatography (FIGURE 6 and FIGURE 6A) shows that exclusion of the above-identified impurities using the Milli-Pore Pellicon cassette system fitted with a 10,000 molecular weight filter pack, has occurred, during processing of the drug of the invention.

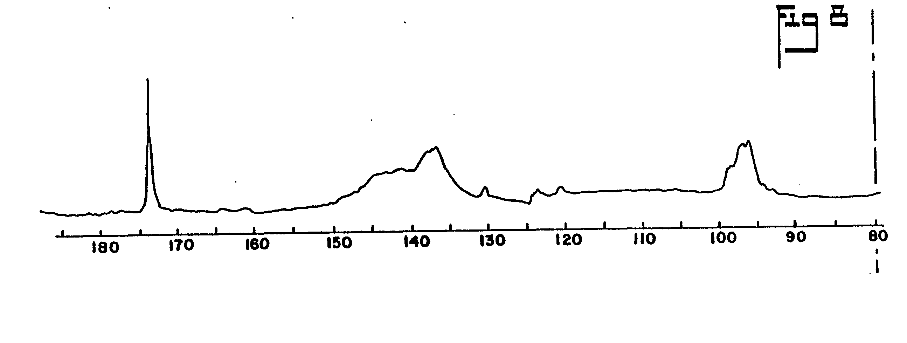

The biologically active drug of this invention is probably an aggregate of ether molecules formed between two hematoporphyrin molecules by linkage of the hydroxyethylvinyl groups as shown in FIGURE 7. This linkage may occur through hydroxyethylvinyl groups in position 3- or 8- as numbered in FIGURE 7. Linkage may be achieved at position 3- in both halves of the ether, at position 8- in both halves of the ether or through position 3- in one half of the ether and in position 8- in the other half of the ether.

These structures may be named as derivatives of ethyl ether, i.e.:

Bis-1-{3-(1-hydroxyethyl)deuteroporphyrin-8-yl} ethyl ether, as shown in FIGURE 7.

Other structured isomers may be named; 1-{3-(1-hydroxyethyl)deuteroporphyrin-8-yl}-1'-{8-(1-hydroxyethyl)deuteroporphyrin-3-yl}ethyl ether, or

1-{8-(1-hydroxyethyl)deuteroporphyrin-3-yl}-1' {3-hydroxyethyl)deuteroporphyrin-8-yl}ethyl ether, and

Bis-1-{8-(1-hydroxyethyl)deuteroporphyrin-3-yl}ethyl ether.

One or both hydroxyethyl groups at positions 3- or 8-, not used in ether formation, may dehydrate to form vinyl groups. Although experiments have not been conducted, experience indicates that ethers as shown in FIGURE 7 might be substituted with various combinations of hydrogen, alkyl groups, carboxylic acid groups and alcohol-containing groups at various locations of the structure. In addition, many possible optical isomers of these structures exist.

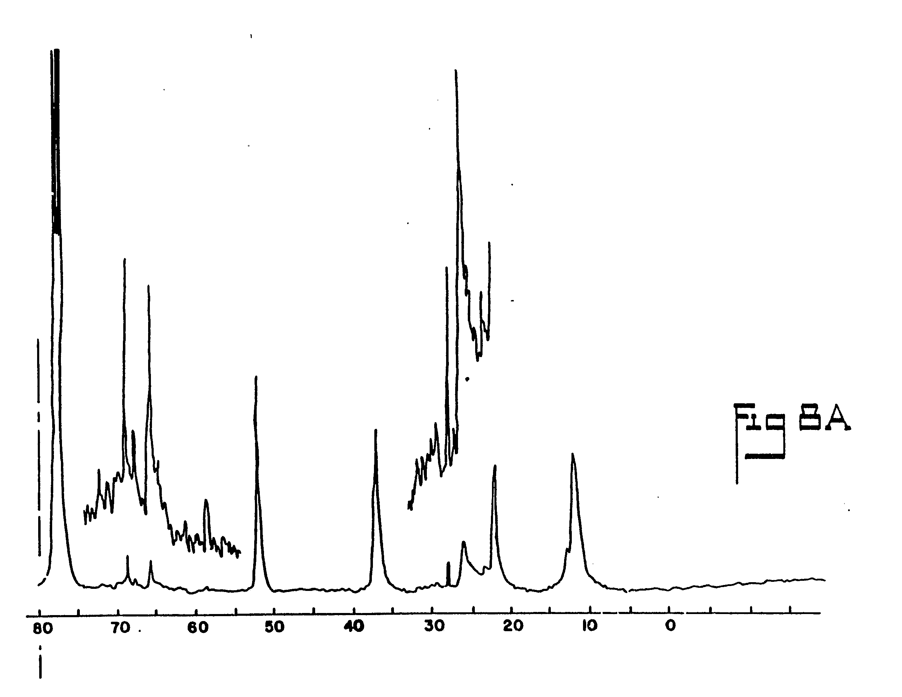

A carbon-13 nuclear magnetic resonance study (FIGURES 8 and 8A) of the drug in deuterated chloroform referenced to tetramethysilane reveals two additional absorbances not previously apparent in FIGURE 4. Peaks at 24.7 ppm and 62 ppm in FIGURE 4 have shifted to 25.9 ppm and 65.3 ppm respectively in FIGURE 8A but newly-developed peaks at 27.9 ppm and 68.4 ppm in FIGURE 8A represent resonances for $CH_3$ and H—$C$—OH bonded from position 3- in FIGURE 8A, respectively. These newly-developed resonances substantiate the molecular formula depicted in FIGURE 7.

While tests using the new drug have been performed to date on animals, it is believed that equivalent results would and will be obtained on humans, utilizing the same or less relative amount of drug to body weight. It is believed that the aforedescribed treatment utilizing the drug of the invention, can be used repeatedly without cumulative damage to normal tissues, providing that treatment is not overly aggressive.

While the aforementioned animal tests utilized a dosage of the new drug of approximately 4 mg/kg of body weight, in the treatment of the tumors in humans, dosages as low as 1 mg/kg of body weight are believed effective utilizing the new drug. In any event dosages of the new drug of only approximately one-half of the necessary prior art dosages of the prior art related porphyrin drug, are equivalently effective in accomplishing necrosis of tumors.

Also, while the aforementioned animal tests utilized illumination one day following injection of the new drug, it is believed that a delay of up to seven days prior to illumination still will accomplish necrosis, and a time delay of two to four days between injection and illumination is generally preferable in humans.

Furthermore, while an intensity of 160 mw/cm$^2$ for 30 minutes was utilized to activate the drug, it is believed that an intensity as high as 4000 mw/cm$^2$ for 20 minutes or as low as 5 mw/cm$^2$ for an extended period of time may be utilized to accomplish necrosis. Less than 5 mw/cm$^2$ of illumination intensity will probably have no therapeutic effect, irrespective of time of application.

From the foregoing description, and accompanying drawings, it will be seen that the invention provides a new and novel drug, useful in the diagnosis and treatment of tumors, permitting utilization of reduced amounts of the drug as compared to related prior art drugs, and which results in less severe side effects.

## Claims

1. A composition of matter effective for localizing and/or destroying tumors, said composition being fluorescent and photosensitive and having the capability of localizing in and being retained in tumor cells as compared to normal tissues, whereby the composition is comprised of high molecular aggregates of a porphyrine derivative, said composition further having a visible spectrum with absorption peaks at 505, 537, 565, and 615 nm, an infrared spectrum with absorption peaks at 3.0, 3.4, 6.4, 7.1, 8.1, 9.4, 12 and 15 microns, a carbon-13 NMR resonance spectrum at 9.0, 18.9, 24.7, 34.5, 62, 94.5, 130—145, and 171.7 ppm relative to a 37.5 ppm resonance for DMSO, and a mass spectrum showing mass numbers of 1899, 1866, 1809, 1290, 1200, 609, 591, 219, and 149 obtainable by

a) reacting hematoporphyrine with acetic/sulfuric acid and precipitating the resulting derivate,

b) adjusting the pH of an aqueous suspension of said derivative to 7 to 7.4 and

c) recovering high molecular aggregates of the derivative by filtration through a membrane system excluding substances with a molecular weight below 10,000.

2. A pharmaceutical composition containing the composition of matter of claim 1 as active ingredient.

3. Pharmaceutical composition of claim 2, characterized in that the active ingredient is present in a concentration of 2.5 mg/cc of aqueous solution.

4. A process for preparing the composition of claim 1 by reacting hematoporphyrine with acetic/sulfuric acids to form a solution of hematoporphyrine derivative and precipitating said derivative from the solution with sodium acetate, characterized in that the hematoporphyrine derivative is subsequently suspended in water, the pH of the aqueous suspension is adjusted to 7.0 to 7.4, and the resulting solution is filtered through a membrane system excluding substances with a molecular weight below 10,000.

5. A process according to claim 4, characterized in that prior to the filtration step sodium chloride is added to the solution in an amount sufficient to render the solution isotonic.

## Patentansprüche

1. Eine Stoff-Zusammensetzung zur Lokalisierung und/oder Zerstörung von Tumoren, wobei die genannte Zusammensetzung fluoreszierend und photosensitiv ist und die Fähigkeit besitzt, Tumorzellen im Vergleich zu normalen Geweben zu lokalisieren und in ihnen zurückgehalten zu werden, wobei die Zusammensetzung hochmolekulare Aggregate eines Porphyrinderivats enthält, die genannte Zusammensetzung ferner ein sichtbares Spektrum mit Absorptionsmaxima bei 505, 537, 565 und 615 nm, ein Infrarotspektrum mit Absorptionsmaxima bei 3,0, 3,4, 6,4, 7,1, 8,1, 9,4, 12 und 15 Mikrometer, ein Kohlenstoff-13-NMR-Resonanzspektrum bei 9,0, 18,9, 24,7, 34,5, 62, 94,5 130—145 und 171,7 ppm, bezogen auf eine Resonanz von 37,5 ppm für DMSO, und ein Massenspektrum mit Bassezahlen von 1899, 1866, 1809, 1290, 1200, 609, 591, 219 und 149 hat, erhältlich durch

a) Umsetzen von Hematoporphyrin mit Essig-/Schwefelsäure und Fällen des resultierenden Derivats,

b) Einstellen des pH einer wäßrigen Suspension des genannten Derivats auf 7 bis 7,4 und

c) Rückgewinnen hochmolekularer Aggregate des Derivats durch Filtration durch ein Membransystem, das Substanzen mit einem Molekulargewicht unter 10 000 ausschließt.

2. Eine pharmazeutische Zusammensetzung, die als Wirkstoff die Stoff-Zusammensetzung nach Anspruch 1 enthält.

3. Pharmazeutische Zusammensetzung nach Anspruch 2, dadurch gekennzeichet, daß der Wirkstoff in einer Konzentration von 2,5 mg/ml wäßriger Lösung vorliegt.

4. Ein Verfahren zur Herstellung der Zusammensetzung nach Anspruch 1 durch Umsetzen von Hematoporphyrin mit Essig-Schwefelsäuren zur Bildung einer Lösung von Hematoporphyrin-derivat und Fällen des genannten Derivats aus der Lösung mit Natriumacetat, dadurch gekennzeichnet, daß das Hematoporphyrinderivat nachfolgend in Wasser suspendiert wird, der pH der wäßrigen Suspension auf 7,0 bis 7,4 eingestellt wird und die resultierende Lösung durch ein Membransystem filtriert wird, das Substanzen mit einem Molekulargewicht unter 10 000 ausschließt.

5. Ein Verfahren nach Anspruch 4, dadurch gekennzeichnet, daß der Lösung vor dem Schritt der Filtration Natriumchlorid in einer Menge zugesetzt wird, die ausreicht, um die Lösung isotonisch zu machen.

**Revendications**

1. Composition d'une matière efficace pour localiser et/ou détruire les tumeurs, ladite composition étant fluorescente et photosensible et possédant la capacité de se localiser et d'être retenue dans les cellules tumorales par comparaison avec les tissus normaux, caractérisée en ce que la composition est composée d'agrégats de haut poids moléculaire d'un dérivé de la porphyrine, ladite composition ayant, en outre, un spectre visible comportant des pics d'absorption à 505, 537, 565 et 615 nm, un spectre infra-rouge comportant des pics d'absorption à 3,0, 3,4, 6,4, 7,1, 8,1, 9,4, 12 et 15 microns, un spectre de résonance RMN du carbone 13 à 9,0, 18,9, 24,7, 34,5 62, 94,5, 130—145 et 171,7 ppm par rapport à une résonance à 37,5 ppm pour le DMSO, et un spectre de masse montrant des nombres de masse de 1899, 1866, 1809, 1290, 1200, 609, 591, 219 et 149, et peut être obtenue par

a) mise en réaction de d'hématoporphyrine avec un mélange d'acide acétique et sulfurique et précipitation du dérivé résultant

b) ajustement du pH d'une suspension aqueuse dudit dérivé à 7 à 7,4 et

c) récupération d'agrégats de haut point moléculaire du dérivé par filtration à travers un système membranaire excluant des substances ayant un poids moléculaire inférieur à 10 000.

2. Composition pharmaceutique contenant la composition de matière de la revendication 1 en tant qu'ingrédient actif.

3. Composition pharmaceutique selon la revendication 2, caractérisée en ce que l'ingrédient actif est présent à une concentration de 2,5 mg/cm$^3$ de solution aqueuse.

4. Procédé de préparation de la composition selon la revendication 1 par mise en réaction de l'hématoporphyrine avec un mélange d'acides acétique et sulfurique pour former une solution d'une dérivé d'hématoporphyrine et précipitation dudit dérivé à partir de la solution avec de l'acétate de sodium, caractérisé en ce que le dérivé d'hématoporphyrine est ultérieurement mis en suspension dans de l'eau, le pH de la suspension aqueuse est ajusté à 7,0 à 7,4 et la solution résultante est filtrée à travers un système membranaire excluant des substances ayant un poids moléculaire inférieur à 10 000.

5. Procédé selon la revendication 4, caractérisé en ce que avant l'étape de filtration, on ajoute du chlorure de sodium à la solution en une quantité suffisante pour rendre la solution isotonique.

Fig 1

Fig 2

Fig 3

EP 0 120 054 B1

Fig 3A

EP 0 120 054 B1

EP 0 120 054 B1

Fig. 4

Fig 5

Fig 5A

Fig 6

Fig 6A

Fig 7

Fig. 8

Fig. 8A